# FASCICULE DE BREVET EUROPEEN

(11) **EP 2 506 723 B1**
(45) Date de publication et mention de la délivrance du brevet: **10.05.2017**
(21) Numéro de dépôt: 10785418.4
(22) Date de dépôt: 30.11.2010
(51) Int. Cl.: A61K 8/97, A61Q 19/00, A61K 36/48, A23C 9/152, A23L 2/52, A23L 2/66, A61Q 3/02, A61Q 7/00, A61Q 17/00, A61Q 17/04, A61Q 19/02, A61Q 19/06, A61Q 19/08, A23L 7/126, A23L 33/18, A23L 33/185, A61Q 5/00, A23L 33/105

(54) **EXTRAIT DE GRAINES D'ACACIA MACROSTACHYA ET COMPOSITIONS LE COMPRENANT**
ACACIA-MACROSTACHYA-SAMENEXTRAKT UND ZUSAMMENSETZUNGEN DAMIT
ACACIA MACROSTACHYA SEED EXTRACT AND COMPOSITIONS CONTAINING SAME

(30) Priorité: 30.11.2009 FR 0958525
(43) Date de publication de la demande: 10.10.2012
(73) Titulaire: Laboratoires Expanscience, 92400 Courbevoie (FR)
(72) Inventeur: MSIKA, Philippe, F-78000 Versailles (FR); SAUNOIS, Alex, F-28210 Nogent-Le-Roi (FR); LECLERE-BIENFAIT, Sophie, F-28100 Dreux (FR); BAUDOIN, Caroline, F-78120 Rambouillet (FR)
(74) Mandataire: Regimbeau
(86) Numéro de dépôt international: PCT/EP2010/068574
(87) Numéro de publication internationale: WO 2011/064402

(56) Documents cités:
- WO-A1-2004/089392
- WO-A1-2005/105123
- WO-A1-2005/115421
- DE-A1-102006 005 767
- FR-A1- 2 778 565
- FR-A1- 2 902 335
- anonymous: "Acacia macrostachya auct.", African plant database , 1 janvier 2009 (2009-01-01), XP002590187, Extrait de l'Internet: URL:http://www.ville-ge.ch/musinfo/bd/cjb/ africa/details.php?langue=an&id=76770 [extrait le 2010-07-01]
- BRAIN P.: "Immunology and phylogeny: a preliminary study on Acacia", SOUTH AFRICAN JOURNAL OF SCIENCE, vol. 83, 1 juillet 1987 (1987-07-01), pages 422-427, XP009135655, South Africa
- MUSTAFA N.K., TANIRA M.O.M. DAR F.K. AND NSANZE H.: "Antimicrobial activity of Acacia nilotica subspp. nilotica fruit extracts", PHARM. PHARMACOL. COMMUN., vol. 5, 1 juillet 1999 (1999-07-01), pages 583-586, XP009135643,
- SERE A, NONGONIERMA A, KANE A, SAMBO G: "Etude d'une plante de la pharmacopée traditionelle: Acacia macrostachya", MEDECINE D'AFRIQUE NOIRE, XES JOURNEES MEDICALES DE DAKAR, COMMUNICATIONS LIBRES, vol. 30, no. 1, 1 janvier 1983 (1983-01-01), pages 29-34, XP009135654,
- ADEWUSI STEVE R A ET AL: "Chemical composition of Acacia colei and Acacia tumida seeds potential food sources in the semi-arid tropics", FOOD CHEMISTRY, ELSEVIER LTD, NL LNKD- DOI:10.1016/S0308-8146(02)00253-4, vol. 80, no. 2, 1 février 2003 (2003-02-01), pages 187-195, XP009135644, ISSN: 0308-8146 [extrait le 2002-11-02]

## Description

L'invention se rapporte à une composition comprenant un extrait de graines d'*Acacia macrostachya*, tel qu'un extrait peptidique et osidique de graines d'*Acacia macrostachya.* La composition est avantageusement cosmétique, pharmaceutique, dermatologique, ou nutraceutique. L'invention a également pour objet un procédé d'extraction d'un extrait de graines d'*Acacia macrostachya,* ainsi que l'extrait susceptible d'être obtenu par ledit procédé. L'invention concerne également une telle composition ou un tel extrait pour son utilisation dans la prévention ou le traitement des troubles ou pathologies de la peau, des muqueuses ou des phanères, pour son utilisation dans la prévention ou le traitement des troubles vasculaires, ou encore pour son utilisation dans la prévention ou le traitement des altérations du tissu adipeux. L'invention concerne enfin un procédé de soin cosmétique de la peau, des phanères ou des muqueuses, en vue d'améliorer leur état ou leur aspect, comprenant l'administration ou consistant à administrer une telle composition ou un tel extrait.

Le genre *Acacia* comprend un grand nombre d'espèces ligneuses bien adaptées aux climats chauds et secs, à très faibles précipitations. Certaines espèces sont largement répandues dans les régions tropicales semi désertiques d'Afrique de l'Ouest, d'Australie, du Pakistan... Beaucoup d'*Acacia* possèdent donc un rôle écologique et quelques uns un rôle économique important : *A. senegal* source de gomme arabique ; *A. catechu* source de tanins ; *A. dealbata* (notre mimosa) horticole aux fleurs ornementales. A côté de ces espèces bien connues en occident, *Acacia macrostachya* (synonyme: *Acacia ataxacantha*) est resté assez marginal. C'est surtout l'usage alimentaire de ses graines qui a permis quelques écrits à son sujet. Les données actuelles le concernant proviennent essentiellement de deux ou trois ouvrages occidentaux sur les plantes d'Afrique sahélienne et de documents d'organisations internationales rédigés par des auteurs pour la plupart burkinabés. Ainsi, cette utilisation traditionnelle est parfaitement décrite dans des rapports de M. Ouétian Bognounou, Officier et Chevalier de l'Ordre National, Chevalier des Palmes académiques, chargé de recherche en floristique et ethnobotanique de l'INERA du Burkina Faso co-écrits avec M. Marc Olivier, consultant en ethnobotanique. *Acacia macrostachya* est une plante très peu connue en dehors des savanes et zones urbaines d'Afrique de l'Ouest, ainsi que çà et là dans d'autres régions sèches où il fait partie du paysage et où la graine est consommée. Aucune partie de cette plante n'est l'objet d'un commerce significatif entre pays. Les médias et les entreprises commerciales des pays occidentaux n'ont pas communiqué jusqu'à présent sur l'*Acacia macrostachya.*

### La plante Acacia macrostachya

De nom botanique *Acacia macrostachya* Rchb. Ex DC., cet arbuste appartient à l'ordre des *Fabales* et à la famille botanique des *Mimosaceae.* Les noms usuels de la plante sont au Burkina Faso : zanmné, karitiga, zamenega, kiese mamonguala (l'arbre qui accroche au passage) ; au Sénégal, 16 noms différents issus de 12 ethnies.

Cet arbuste sarmenteux et épineux de 2 à 5 mètres de haut peut occasionnellement devenir un arbre atteignant 8 m, à cime étroite et ouverte. Son écorce est souvent fissurée, fibreuse, grise à tranche striée de blanc. Il possède des rameaux bruns, pubescents, pourvus tout le long de minces épines dispersées et crochues d'un cm de longueur. Les tiges sont longues et flexibles.
Ses feuilles vert tendre, alternes, bipennées, sont disposées toutes dans le même plan. Elles comprennent un pétiole de 8 à 22 cm de long, pubescent et épineux au dessous, portant une glande ronde allongée avant la première paire de pinnule. Chaque pétiole porte 20 à 30 paires de pinnule (pétiolule) avec 20 à 50 paires de foliolules par pinnule. Les foliolules sont courtes, pubescents, linéaires et asymétriques.
L'inflorescence épineuse, est formée de 1 à 2 épis cylindriques, de 7 à 8 (5-12) cm de long, aux nombreuses fleurs disposées à l'aisselle des feuilles, de couleur crème devenant jaunâtre. Le nectar et le pollen des fleurs sont butinés par les abeilles. Acacia présente une haute valeur mellifère. La floraison se produit en fin de saison sèche ou en début de saison des pluies après la feuillaison.

Le fruit est une gousse mince aplatie et légèrement ondulée, oblongue, (7 à 15 x 1,5 à 2,0 cm), acuminé aux deux extrémités, brun rouge à maturité. Il contient 7 à 8 graines (2-3 graines selon A. Sere et al., 1983). Ces dernières sont brunes, rondes et aplaties. Elles mesurent 7 à 8 mm de diamètre. Le poids des graines est d'approximativement 77 g pour 1000 graines.

### Caractéristiques de la graine

Les données de la littérature relatives à la composition chimique des graines d'*Acacia macrostachya* sont presque inexistantes.
Les graines seraient, parait-il, très riches en protéines, fer et vitamines C. Un résumé article rédigé en français (Parkouda et al., 2006) concerne l'évaluation de la qualité nutritionnelle (glucides, lipides, protéines et minéraux) d'espèces fruitières du Burkina Faso dont *Acacia macrostachya,* mais n'en précise pas de teneurs.

Des analyses nutritionnelles réalisées par la Demanderesse sur des graines de différentes origines montrent qu'elles peuvent contenir par exemple :
- 38% de fibres
- 38% de protéines
- 8% de lipides
- 2.5% de saccharose

### ART ANTERIEUR

### Usage médicinal :

L'*Acacia macrostachya* est mentionné comme étant une plante médicinale en Guinée et au Sénégal (Penso, 2001), ainsi qu'au Mali (Boullard, 2001). Cependant, la graine semble très peu employée à des fins thérapeutiques.

Le zaméné (graine bouille lavée et cuite) soignerait les maux de ventre et serait apprécié par les personnes souffrant d'hypertension artérielle.

### Observations complémentaires :

En médecine populaire sahélienne notamment au Sénégal, l'écorce (Arbonnier, 2002; Neuwinger, 1996) ou les jeunes feuilles bouillies (Neuwinger, 1996) sont utilisées dans les troubles gastro-intestinaux en cas de flatulence, vomissements et de diarrhées.

L'écorce est aussi employée comme désinfectante et anthelminthique (Arbonnier, 2002; Neuwinger, 1996). Les racines sont indiquées en cas de blennorragie et de syphilis (Arbonnier, 2002).

En Gambie, les racines macérées sont employées lors de désordres stomacaux (Neuwinger, 1996). Les feuilles en usage externe ou interne constitueraient un antidote du venin des serpents (Arbonnier, 2002).

Au Mali, écorces des racines et feuilles sont préconisées pour calmer les maux de ventre des enfants, les dysenteries et les diarrhées (Boullard, 2001).

En Casamance, au Mali et au Burkina Faso, la racine est préconisée par les tradipraticiens à des fins aphrodisiaques (Sere et al., 1983).

Au Burkina Faso, la décoction des feuilles est appliquée localement pour soigner les abcès dentaires (Tapsoba et Deschamps, 2006).

### Usage dermatologique et cosmétique :

Aucun usage courant n'a été à ce jour rapporté.

### DESCRIPTION DE L'INVENTION

La Demanderesse a découvert que les extraits de graines d'*Acacia macrostachya* présentent des propriétés cosmétiques et dermatologiques jamais décrites jusqu'à présent. En particulier, c'est la première fois que des extraits d'*Acacia macrostachya* sont utilisés en tant que tels, pour leurs propriétés spécifiques.

L'invention a pour objet une composition comprenant un extrait de graines d'*Acacia macrostachya,* ledit extrait de graines comprenant des peptides et/ou des sucres, avantageusement un mélange de peptides et de sucres, éventuellement en association avec un excipient approprié.

La composition est avantageusement cosmétique, pharmaceutique, dermatologique ou nutraceutique. Ladite composition est de préférence formulée pour être administrée par voie topique externe ou orale.

L'extrait de graines d'*Acacia macrostachya* selon l'invention est un extrait peptidique et osidique.

Par « extrait peptidique et osidique », on entend un extrait comprenant majoritairement ou essentiellement des peptides et des oses (sucres).

Avantageusement, l'extrait de graines d'*Acacia macrostachya* est constitué essentiellement d'un mélange de peptides et de sucres.

L'extrait de graines d'*Acacia macrostachya* selon l'invention est substantiellement débarrassé de toute protéine native résiduelle, car ces protéines peuvent provoquer des réactions allergiques que l'on souhaite éviter.

Typiquement, l'extrait de graines d'*Acacia macrostachya* est substantiellement débarrassé des acides aminés libres.

L'extrait selon la présente invention comprend avantageusement 10 à 90 % en poids de peptides et 10 à 90 % en poids de sucres totaux, les pourcentages étant exprimés par rapport au poids total dudit extrait.

Dans un mode de réalisation particulier de la présente invention, l'extrait comprend 20 à 70 %, avantageusement 20 à 60 %, avantageusement 30 à 65 %, typiquement 30 à 55 %, en particulier 45 à 50%, en poids de peptides

Dans un autre mode de réalisation particulier de la présente invention, l'extrait comprend 20 à 60 %, avantageusement 30 à 55 %, en particulier 45 à 50%, en poids de sucres.

Selon un aspect préféré de l'invention, l'extrait peptidique et osidique comprend 50 % en poids de peptides et 45 % en poids de sucres, les pourcentages étant exprimés par rapport au poids total de matière active dudit extrait avant ajout par exemple d'un éventuel support de séchage.

Selon une caractéristique particulière de la présente invention, le ratio peptides/sucres de l'extrait est supérieur à 0,75, et avantageusement compris entre 1 et 2.

Selon une variante avantageuse de l'invention, la composition contient 0,001 à 10 %, typiquement 0,01 à 5 %, en poids d'extrait, exprimé en pourcentage d'extrait sec.

L'invention a également pour objet un procédé de préparation d'un extrait peptidique et osidique de graines d'*Acacia macrostachya* substantiellement débarrassé de toute protéine native résiduelle.

Avantageusement selon l'invention, le procédé de préparation d'un extrait peptidique et osidique de graines d'*Acacia macrostachya* comprend les étapes successives suivantes :
- broyage des graines d'*Acacia macrostachya,*
- dispersion des graines d'*Acacia macrostachya* broyées dans de l'eau ou en phase aqueuse, avantageusement à un pH compris entre 3.0 et 9.0 et à une température comprise entre 20 et 90°C,
- hydrolyse enzymatique de ladite dispersion, et
- récupération de l'extrait peptidique et osidique.

Avant d'être dispersées, les graines broyées peuvent être délipidées, notamment dans de l'éthanol.

Selon l'invention, suite à la dispersion en phase aqueuse, on réalise une hydrolyse de ladite dispersion, telle qu'une hydrolyse enzymatique.

Typiquement, l'hydrolyse enzymatique est réalisée par une ou des enzymes adaptées dans les conditions optimales de pH et de température, connues de l'homme de l'art, par exemple à un pH compris entre 3.0 et 9.0 et typiquement à une température comprise entre 20 et 90°C, avantageusement par au moins une carbohydrase telle qu'une pectinase ou cellulase, ou par un mélange de protéases et de carbohydrases, telles que des pectinases, cellulases, arabanases, hémicellulases, xylanases et β-glucanases, puis on récupère l'extrait peptidique et osidique.

Selon un aspect avantageux de l'invention, l'hydrolyse est une hydrolyse enzymatique par au moins une protéase ou une carbohydrase.

L'hydrolyse enzymatique de la dispersion peut être suivie si besoin d'un traitement thermique afin de dénaturer les enzymes, typiquement entre 80 et 100°C.

L'étape d'hydrolyse du procédé selon l'invention est très importante, puisqu'elle permet de transformer ou de « découper » les protéines natives présentes dans les graines d'Acacia en peptides. Cette étape permet également avantageusement de transformer ou de « découper » les polysaccharides présents dans les graines d'Acacia en oligosaccharides ou monosaccahrides.

De manière particulièrement avantageuse selon l'invention, le procédé comprend une étape d'hydrolyse telle qu'une hydrolyse enzymatique, puis une étape d'ultrafiltration par exemple à un seuil de coupure compris entre 10000 Daltons et 15000 Daltons, pour éliminer les protéines résiduelles qui sont potentiellement allergisantes et éventuellement les enzymes.

Dans un mode de réalisation particulier selon l'invention, le procédé comprend également une étape de nanofiltration avec par exemple un seuil de coupure compris entre 100 Daltons et 300 Daltons, avantageusement entre 130 et 300 Daltons, typiquement entre 200 Daltons et 300 Daltons, pour éliminer les acides aminés libres ou les sels minéraux, suite à l'étape d'ultrafiltration.

Selon une variante avantageuse de l'invention, suite à l'hydrolyse de la dispersion et préalablement à la récupération de l'extrait peptidique et osidique, on procède à une filtration ou à une centrifugation, éventuellement suivie d'une ultrafiltration, diafiltration, et/ou nanofiltration.

Préférentiellement, à titre d'exemple, l'extrait peptidique et osidique peut être obtenu selon le procédé suivant :
a) délipidation des graines préalablement broyées par dispersion à 10% de matière sèche dans de l'éthanol à 96% ;
b) après élimination de l'éthanol, remise en solution des graines ainsi délipidées à 10% de matière sèche dans l'eau ;
c) hydrolyse enzymatique des carbohydrates par action combinée d'une pectinase / cellulase (Peclyve LI de la société Lyven par exemple) et d'un autre mélange de carbohydrases à activités complémentaires, telles que arabanase, β glucanase, hémicellulase, xylanase (Viscozyme L de la société Novozymes par exemple) dans les conditions de pH et température optimales pour l'activité de ces enzymes ;
d) suivie d'une hydrolyse par une protéase alcaline (par exemple la Prolyve 1000 de la société Lyven) ;
e) traitement thermique afin de dénaturer les enzymes ;
f) centrifugation, ultrafiltration et/ou diafiltration sur membranes 15 kDa afin d'éliminer les protéines résiduelles potentiellement allergisantes;
g) nanofiltration sur membrane 200 Da afin d'éliminer des sels minéraux ou des acides aminés libres par exemple ;

Avantageusement selon l'invention, suite à la récupération de l'extrait peptidique et osidique, on procède à au moins l'une des étapes suivantes :
- décoloration de l'extrait ainsi obtenu, par exemple en présence de charbon actif ou par tout autre moyen connu de l'homme de l'art, et
- séchage de l'extrait obtenu sur un support ou sans support.

Avantageusement, l'extrait peptidique et osidique peut être séché par des procédés connus de l'homme de l'art, en présence ou non d'un support de type, par exemple, maltodextrines ou fibres d'acacia (Fibregum® société CNI) ; typiquement selon un ratio pouvant varier de 0% à 80% de support par rapport au pourcentage de matière sèche obtenu dans la forme liquide de l'extrait et préférentiellement séché par lyophilisation afin d'obtenir dans la poudre finale, 50% de matière sèche issue de l'extrait et 50% de support de lyophilisation.

### Exemple d'extrait peptidique et osidique liquide ainsi obtenu:

### 1 - Analyse physico-chimique (% / matière sèche totale)

| | |
|---|---|
| Extrait sec (2h, 105°C, étuve ventilée) | : 6,5% |
| pH | : 4.0 |
| Azote α-aminé (OPA, équivalent leucine) | : 24% |
| Peptides (Kjeldahl, N x 6.25) | : 47% |
| Sucres solubles (HPLC) | : 45% dont typiquement du glucose, fructose et saccharose |
| Cendres totales | : 16% |
| Polyphénols (Folin-Cioccalteu, en acide gallique) | : 3% |

L'extrait selon l'invention contient ainsi typiquement des polyphénols.

### 2 - Profil des répartitions des masses molaires des peptides solubles

| | |
|---|---|
| Inférieur à 130 Da : | 20% |
| Entre 130 - 300 Da : | 17% |
| Entre 300 - 1200 Da : | 48% |
| Entre 1200 - 3500 Da : | 14% |
| Supérieur à 3500 Da : | ≤1% |

La présente invention a également pour objet un extrait de graines d'*Acacia macrostachya* substantiellement débarrassé de toute protéine native résiduelle susceptible d'être obtenu par le procédé mentionné ci-dessus. Un tel extrait contient avantageusement 10 à 90 % en poids de peptides et 10 à 90 % en poids de sucres.

Dans un mode de réalisation particulier de la présente invention, l'extrait comprend 20 à 70 %, avantageusement 20 à 60 %, avantageusement 30 à 65 %, typiquement 30 à 55 %, en particulier 45 à 50%, en poids de peptides

Dans un autre mode de réalisation particulier de la présente invention, l'extrait comprend 20 à 60 %, avantageusement 30 à 55 %, en particulier 45 à 50%, en poids de sucres.

Typiquement, l'extrait selon la présente comprend des monosaccharides ou oligosaccharides, tels que du glucose, du fructose, du saccharose, ou leurs mélanges. En particulier, des sucres de l'extrait sont composés majoritairement de glucose, de fructose et de saccharose

Selon une caractéristique particulière, le ratio peptides/sucres de l'extrait selon l'invention est supérieur ou égal à 0,75, et avantageusement compris entre 1 et 2.

Avantageusement selon l'invention, les peptides de l'extrait présentent une masse moléculaire inférieure ou égale à 3500 Dalton. En particulier, les peptides de l'extrait présentent majoritairement une masse moléculaire inférieure ou égale à 1200 Dalton, typiquement entre 300 et 1200 Dalton.

Typiquement, l'extrait peptidique et osidique selon l'invention ne contient substantiellement pas de protéines résiduelles qui sont potentiellement allergisantes.

Dans un mode de réalisation particulier, l'extrait peptidique et osidique selon l'invention ne contient substantiellement pas d'acides aminés libres.

Selon un autre aspect de l'invention, la composition peut en outre comprendre au moins un autre composé actif en plus de l'extrait de graines d'*Acacia macrostachya.*

Cet autre composé peut être choisi parmi tous les composés et leurs équivalents fonctionnels, énoncés ci-dessous.

Cet autre composé peut être en particulier choisi parmi des actifs classiquement utilisés en dermatologie ou cosmétique tels que les émollients, les actifs hydratants, les kératorégulateurs, les kératolytiques, les agents restructurant de la barrière cutanée, des agonistes PPARs (ou Peroxysome Proliferator Activated Receptor), les agonistes RXR ou LXR, les agonistes des récepteurs à la vitamine D ou aux corticoïdes, les activateurs de la différenciation des kératinocytes (rétinoïdes, calcidone®, le calcium), les agents sébo-régulateurs, les agents anti-irritants, les agents apaisants, les agents anti-inflammatoires, les agents anti-oxydants et les agents anti-âge.

Cet autre composé peut aussi être choisi parmi des principes actifs ayant une action thérapeutique ou cosmétique complémentaire, tels que les antibiotiques, les pré et probiotiques, les agents anti-bactériens, les composés antifongiques, les agents anti-viraux, les conservateurs, les immunomodulateurs (tacrolimus ou pimécrolimus), les oxazolines, les facteurs de croissance, les agents cicatrisants ou les molécules eutrophiques, les médicaments, les agents pigmentants ou hypopigmentants, les agents lipolytiques ou inhibiteurs de la lipogénèse ou encore les agents anti-cellulite ou amincissants, les filtres et écrans solaires minéraux ou organiques (pigmentaires ou ultrafins), des aliments classiques ou fonctionnels : hyper ou hypoglycémiants, des nutriments anti-graisse ou anti-cellullite, anti-cholestérol, anti-oxydant, énergisant, reconstituant, ou ayant un impact sur les signes secondaires de la ménopause.

Cet autre composé peut aussi être choisi parmi des extraits naturels de plante (parties de végétaux extractibles en phase aqueuse ou huileuse : polyphénols, flavonoides, autres peptides et sucres, .....), des composés contenant des insaponifiables d'huiles végétales, des insaponifiables stéroliques ou des produits pouvant en contenir (insaponifiables d'huiles végétales, notamment insaponifiables d'huile de Soja, insaponifiables de beurres végétaux ou de matières butyreuses et leurs mélanges, insaponifiables de cires naturelles, insaponifiables d'extraits huileux, insaponifiables de co-produits huileux industriels, insaponifiables d'extraits de corps gras d'origine animale, insaponifiables d'huiles marines, insaponifiables d'extraits de la matière grasse lactique, insaponifiables de lipides extraits d'organismes unicellulaires, insaponifiables des lipides extraits des algues et organismes marins, etc), des stérols, des stanols, des phytostérols, des phytostanols, des tocophérols, des concentrats d'huiles de Tournesol, de Colza et/ou de Palme, des oligo-éléments, des vitamines, des acides gras en oméga 3, 6 ou 9, des plantes hypoglycémiantes ou hyperglycémiantes ou sucrantes.

Les actifs hydratants / émollients les plus utilisés sont la glycérine ou ses dérivés, l'urée, l'acide pyrrolidone carboxylique et ses dérivés, l'acide hyaluronique de tout poids moléculaire, les glycosaminoglycanes et tout autres polysaccharides d'origine marine, végétale ou biotechnologique comme par exemple la gomme xanthane, le fucogel^{®}, certains acides gras comme l'acide laurique, l'acide myristique, les acides gras poly- et mono-insaturés de type oméga 3, 6 et 7, 9 comme l'acide linoléique et acide palmitoléique, l'oléodistillat de Tournesol, les peptides d'Avocat, le beurre de Cupuaçu. Les modulateurs de la différenciation épidermique protéines clés du *stratus corneum* ou *granulosum,* pouvant être utilisés en association sont avantageusement les rétinoïdes, les peptides de Lupin, les sucres d'Avocat, ou un extrait peptidique de Quinoa.
Les agents anti-inflammatoires/anti-irritants, apaisants les plus classiques sont l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, les flavonoïdes (thé vert, quercétine...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide^{®} (dérivé d'oxazoline), les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (*Lycium barbarum*), les peptides ou complexes d'acides aminés végétaux ou encore la disulone topique, ou les médicaments anti-inflammatoires stéroïdiens (AIS), tels que les corticoïdes, ou non-stéroïdiens (AINS).

Parmi les agents kératorégulateurs / kératolytiques les plus utilisés se trouvent : les acides de fruits alpha hydroxyacide - AHA (acide citrique, acide glycolique, acide malique, acide lactique...), les esters d'AHA, les associations d'AHA avec d'autres molécules comme l'association acide malique et protéines d'amandes (keratolite®), l'association acide glycolique ou acide lactique avec l'arginine ou encore l'association d'hydoxy-acide avec des molécules lipidiques comme le LHA^{®} pour lipo-hydroxyacide, les complexes d'hydroxyacide amphotères - AHCare, l'acide azélaïque et ses sels et esters, l'écorce de saule (*Salix alba bark* extract), l'acide salicylique (beta hydroxyacide - BHA), et ses dérivés comme l'acide capryloyl salicylique ou en association avec d'autres molécules comme l'association acide salicylique et polysaccharide, le tazarotène, l'adapalène, ainsi que les molécules de la famille des rétinoïdes tels que : la trétinoïne, le rétinaldéhyde, l'isotrétinoïne, ou le rétinol.

Les agents sébo-régulateurs pouvant être utilisés en association sont avantageusement choisis dans le groupe constitué par les inhibiteurs de 5-alpha réductase comme par exemple l'actif 5-alpha Avocuta^{®}. Le zinc (et ses sels gluconate, salicylate et acide pyroglutamique) présente aussi une activité sébo-suppresseur. On peut citer aussi la spironolactone, anti-androgène et antagoniste de l'aldostérone, qui engendre une réduction significative du taux de sécrétion de sébum. D'autres molécules telles que par exemple *Cucurbita pepo,* extraite des graines de citrouille, et l'huile de pépins de courge, le sabal, limitent la production de sébum par inhibition de la 5α-réductase. D'autres séborégulateurs d'origine lipidique agissant sur la qualité du sébum, comme l'acide linoléique, présentent un intérêt.

Les facteurs de croissance pouvant être utilisés en association sont avantageusement la becaplermine et le TGF-beta, l'EGF, le NGF, le VEGF.

On entend par agent antioxydant une molécule qui diminue ou empêche l'oxydation d'autres substances chimiques. Les antioxydants pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'extrait de ginkgo biloba, de calendula, le Cyclocéramide^{®} (dérivé d'oxazoline), les peptides d'Avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le BHT pour butylhydroxytoluène, le BHA pour butylhydroxyanisol, le lycopène ou la lutéine, le beta-carotène, la grande famille des polyphénols comme les tanins, les acides phénoliques, les anthocyanes, les flavonoïdes avec par exemple les extraits de thé vert, de fruits rouges, de cacao, de raisin, de *Passiflore incarnata*, de *Citrus* ou encore les isoflavones comme par exemple la génistéine/génistine, la daidzéine/daidzine. Dans le groupe des anti-oxydants, on trouve aussi les substances anti-glycation, telles que la carnosine ou certains peptides, la n-acetyl-cystéine, ainsi que les enzymes antioxydants ou antiradicalaires comme la SOD (super oxyde dismutase), la catalase, la glutathion peroxydase, la thioredoxine reductase et leurs agonistes.

Les agents cicatrisants et restructurants de la barrière cutanée, permettant de stimuler la synthèse des lipides clés de l'épiderme, et pouvant être utilisés en association sont avantageusement la vitamine A, le panthénol (vitamine B5), les sucres d'Avocat, le Lupéol, l'extrait peptidique de Maca, un extrait peptidique de Quinoa, l'arabinogalactane, l'oxyde de zinc, le magnésium, le silicium, l'acide madécassique ou asiatique, le sulfate de dextran, le coenzyme Q10, la glucosamine et ses dérivés, la chondroïtine sulfate et globalement les glucosaminoglycanes ou GAG, le sulfate de dextran, les céramides, le cholestérol, le squalane, les phospholipides, les peptides de soja fermenté ou non, les peptides végétaux, les polysaccharides marins, végétaux ou biotechnologiques comme les extraits d'algues ou l'extrait de fougère, les oligo-éléments, les extraits de plantes à tanin comme les tanins dérivant de l'acide gallique appelés tanins galliques ou encore hydrolysables, initialement trouvés dans la noix de galle, et les tanins catéchiques résultant de la polymérisation d'unités flavaniques dont le modèle est fourni par le Cachou (*Acacia catechu*). Les oligo-éléments pouvant être utilisés sont avantageusement choisis dans le groupe constitué par le cuivre, le magnésium, le manganèse, le chrome, le sélénium, le silicium, le zinc et leurs mélanges. Peuvent également être utilisés des concentrats de Tournesol, plus avantageusement des concentrats de Tournesol linoléiques, tels que l'actif commercialisé par les Laboratoires Expanscience, Soline^{®}, des insaponifiables d'huile végétale, tel que l'Avocadofurane®, ou des agonistes PPARs (rosiglitazone, pioglitazone), RXR, LXR.

Les agents anti-âge pouvant agir sur les sujets d'âge mûr sont des agents antioxydants et en particulier la vitamine C, ou encore la vitamine A, le rétinol, le rétinal, l'acide hyaluronique de tout poids moléculaire, l'Avocadofurane^{®}, les peptides de Lupin, l'extrait peptidique de Maca.

Les composés antifongiques pouvant être utilisés en association sont avantageusement l'econazole et le ketoconazole.

Les conservateurs antiseptiques pouvant être utilisés en association sont par exemple le triclosan, la chlorhéxidine, les ammoniums quaternaires.

Les antibiotiques pouvant être utilisés en association sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés en association sont avantageusement l'acyclovir et le valacyclovir. Les conservateurs pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glycéryl caprylate, tels que l'hexanediol, et le sodium levulinate, les dérivés de zinc et de cuivre (gluconate et PCA), la phytosphingosine et ses dérivés, le peroxyde de benzoyle, la piroctone olamine, le pyrithione zinc, le sulfure de sélénium.

Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché.

A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, le benzophénone, l'éthylhexyl méthoxycinnamate, l'éthylhexyl diméthyl PABA, le diéthylhexyl butamido triazone.

Les agents amincissants pouvant être utilisés en association sont avantageusement la caféine, le fucus, les extraits végétaux comme par exemple : l'extrait de lierre, de cacao, de guarana, de petit houx, de thé vert, de maté, de poivre de sichuan, de marron d'inde. La *Centella asiatica,* la carnithine, la glauscine, l'escine, l'extrait de petit houx (*Ruscus esculentus*), les isoflavones comme par exemple la génistéine, le Ginko biloba, la forskoline, le rétinol et autres rétinoïdes, la phloridzine, la criste marine peuvent également être utilisé en association.

Les agents anti-chute des cheveux et/ou fortifiants pour les cheveux et les ongles sont avantageusement les phytostérols, les isoflavones comme par exemple les isoflavones de soja, le RTH16^{®}, l'aminexil^{®}, le minoxidil^{®}, le rétinol, le zinc et ses dérivés, la neoruscine, la vitamine E, la vitamine B2, la vitamine B3, la vitamine B6, la vitamine PP, la vitamine B5 (panthénol, bépanthène), la vitamine B8 (vitamine H ou biotine), la vitamine B9 (acide folique), l'alpha hydroxyacide, la quinine, certains acides aminés comme la cystéine, la cystine, la méthionine. Les inhibiteurs de 5-alpha réductase tels que par exemple le finastéride, le dutastéride, *Serenoa serrulata* ou *repens*, l'extrait de *Cucurbita pepo* ou encore /certains phytostérols, peuvent également être utilisés en association. La kératine, les oligoéléments, ou les sels minéraux peuvent également être utilisés en association. Certains extraits protéiques ou lipidiques végétaux comme par exemple les extraits de pfaffia, de sauge, de citron, de ginseng, de quinquina, de jojoba, de marron d'inde, de miel, de blé, d'ortie, d'échinea, de noix de coco peuvent également être utilisés en association.

Les agents anti-pelliculaires (cuir chevelu) sont avantageusement l'extrait de capucine, la vitamine F, le thymol, l'argile, le pyrithione de zinc, le zinc-PCA, le gluconate de zinc, le sulfate de zinc, le camphre, l'extrait de myrte, l'acide salicylique, la vitamine B5, le climbazole, l'ichtyol, le sélénium et ses dérivés, l'extrait de Curbicia, l'extrait de Carthame, l'extrait d'huile de Melaleuca, l'huile de Bourrache et de *Mimosa Tenuiflora*, la propolis, Kertyol, l'acide glycolique, le kéluamid, la cyclopiroxolamine, le piroctone olamine, le capryloyl glycine, le 5 alpha Avocuta.

Les médicaments ou agents cosmétiques pouvant être utilisés en association sont avantageusement les médicaments ou les agents cosmétiques appropriés pour une administration par voie topique ou orale, en particulier pour la prévention et/ou le traitement de l'atopie/eczéma (les corticostéroïdes comme l'hydrocortisone, le desonide, l'acetonide de fluocinolone, le propionate de fluticasone, les immunomodulateurs topiques inhibiteurs de la calcineurine comme le tacrolimus et le pimecrolimus, la cyclosporine, l'azathioprine, le méthotrexate, la vitamine B12, les molécules anti-microbiennes, les anti-histaminiques comme l'hydroxyzine et la diphenhydramine, les antibiotiques, les pré- et pro-biotiques, la naltrexone, les agonistes de PPAR alpha tels que l'oléodistillat de Tournesol, les émollients contenant des céramides ou autres lipides clés épidermiques), de l'acné (les antibiotiques, le péroxyde de benzoyle, les rétinoïdes, l'acide azélaïque, la vitamine PP, la vitamine B3, le zinc, les cyclines), de la rosacée (le perméthol, la génistéine, l'esculoside, le sulfate de dextran, l'hespéridine méthylchalcone, les rétinoïdes, la licochalcone, l'oxyméthazoline, la kinétine, l'extrait de réglisse, les polyphénols, les flavonoïdes, les procyanidols (thé vert..), la vitamine P like, l'extrait de petit houx, le *Sophora japonica*, l'extrait d'Hamamélis, les antibiotiques tels que la doxycycline) ou du psoriasis (les corticoïdes, le calcipotriol, le calcitriol, le tazarotène, l'huile de cade, l'acitrétine, la PUVA thérapie).

Les immunodulateurs pouvant être utilisés en association sont avantageusement le tacrolimus, le pimécrolimus et les oxazolines. Les oxazolines pouvant être utilisées en association sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide^{®}.

Les agents hypopigmentants ou dépigmentants pouvant être utilisés en association sont l'hydroquinone et ses dérivés, l'arbutine, l'acide rétinoïque, le rétinol, le rétinaldéhyde, la trétinoine, l'hydroquinone, les corticoïdes, l'acide kojique, l'acide azélaïque, l'acide ellagique, l'acide pyruvique, l'acide glycolique, la vitamine B3 (niacinamide) ou PP, la vitamine C, le Cyclocéramide^{®}, les dérivés du résorcinol, le resvératrol, des extraits de réglisse ou de murier blanc, l'acide alpha-lipoïque, l'acide linoléique, l'indométhacine, des chélateurs de cations tels que l'EDTA (acide éthylène diamine tétra acétique), les extraits de soja tels que la génistéine. On peut également citer le Sepiwhite® (N-undecylenoyl-L-phénylalanine) commercialisé par la société Seppic, qui est un agent cosmétique dépigmentant.

Les agents pigmentants pouvant être utilisés en association sont par exemple des agents qui colorent la peau comme le dihydroxyacétone et les mélanines ; des agents qui stimulent le procédé de pigmentation naturelle comme les psolarènes ayant des propriétés thérapeutiques en dermatologie (le 8-méthoxypsolarène, le 5-méthoxypsolarène, le 4,5',8-triméthylpsolarène ou des extraits végétaux de *Psorelea corylifolia* et de *Ammi majus*), les caroténoïdes (le lycopène, la canthaxanthine), les agents stimulant la voie de l'AMP cyclique (1. les analogues de l'AMPc, tels que le 8-bromo-AMPc ou le dibutiryl-AMPc, 2. la forskoline, 3. l'isobutyl-méthyl-xanthine ou la théophylline), les activateurs des protéines kinase C (les diacylglycérols, en particulier l'oléyl-acétyl-glycérol), les diols aliphatiques ou cycliques (le 1,2-propandiol, le 5-norbomane-2,2-diméthanol, le norbomane-2,2-diméthano), les diols bicycliques monoterpène, les dérivés de tyrosine (la L-tyrosine, la L-DOPA), le diméthylsulfoxyde, les agents lysomotropiques, les dinucléotides thymidine, les fragments d'ADN, les analogues de l'hormone stimulant les mélanocytes, le 3-isobutyl-1-méthylxanthine, les donneurs d'acide nitrique (Brown, Journal of photochemistry and photobiology B :biology 63 (2001) 148-161) ; ou encore des extraits végétaux comme les peptides de riz, et les algues, démontrant une activité promélanogène : *Laminaria digitata* (Thalitan^{®} de Codif).

Une association particulièrement avantageuse selon l'invention est une composition comprenant l'extrait peptidique et osidique de graines d'*Acacia macrostachya* et des insaponifiables végétaux et animaux, comme par exemple, les insaponifiables d'Avocat et de Soja, et des concentrats d'huile végétale ou animale en insaponifiables, comme par exemple le concentrat d'huile de Tournesol ou d'huile de Palme, ou encore des huiles végétales contenant des insaponifiables comme par exemple les huiles de Soja et de Colza, et les dérivés d'insaponifiables comme les furanes d'Avocat, les insaponifiables stéroliques, les esters de stérols et les dérivés vitaminiques. Les insaponifiables « stéroliques » sont des insaponifiables dont la teneur en stérols, en méthylstérols et en alcools triterpèniques est comprise entre 20 et 95 % en poids, de préférence 45-65 % en poids, par rapport au poids total de l'insaponifiable.

Une association particulièrement avantageuse selon l'invention est une composition comprenant l'extrait de graines d'*Acacia macrostachya* et des sucres d'Avocat (voir demande WO 2005/115421). Cette composition est particulièrement adaptée pour le traitement de la réparation de la barrière cutanée et de l'inflammation.

Une association particulièrement avantageuse selon l'invention est une composition comprenant l'extrait de graines d'*Acacia macrostachya* et des peptides d'Avocat (voir demande internationale WO2005/105123). Cette composition est particulièrement adaptée pour le traitement de l'irritation et de l'inflammation.

Une autre association particulièrement avantageuse selon l'invention est une composition comprenant l'extrait de graines d'*Acacia macrostachya* et une huile d'Avocat (voir les demandes internationales WO2004/012496, WO2004/012752, WO2004/016106, WO2007/057439).

Une autre association particulièrement avantageuse selon l'invention est une composition comprenant l'extrait de graines d'*Acacia macrostachya* et l'Avocadofurane^{®} (furanes d'avocat, pouvant être obtenus par le procédé décrit dans la demande internationale WO 01/21605). Cette composition est particulièrement adaptée pour le traitement de l'inflammation, pour favoriser la cicatrisation, et pour ses propriétés anti-âge.

Une autre association particulièrement avantageuse selon l'invention est une composition comprenant l'extrait de graines d'*Acacia macrostachya* et le 5 alpha Avocuta® (butyl avocadate). Cette composition est particulièrement adaptée pour inhiber la 5-alpha réductase (voir WO 01/52837 et WO 02/06205) et réguler la sécrétion séborrhée se trouvant augmentée dans l'acné ou les pellicules.

Une autre association particulièrement avantageuse selon l'invention est une composition comprenant l'extrait de graines d'*Acacia macrostachya* et les insaponifiables d'Avocat et de Soja. Les insaponifiables d'Avocat et de Soja pouvant être utilisés en association sont avantageusement un mélange d'insaponifiables d'Avocat furaniques et d'insaponifiables de Soja, dans un rapport respectif d'environ 1/3-2/3. Les insaponifiables d'Avocat et de Soja sont encore plus avantageusement le produit Piasclédine^{®}, commercialisé par les Laboratoires Expanscience.

Une autre association particulièrement avantageuse selon l'invention est une composition comprenant l'extrait de graines d'*Acacia macrostachya* et un oléodistillat de Tournesol, encore plus avantageusement avec des concentrats de Tournesol linoléiques, tels que l'actif commercialisé par les Laboratoires Expanscience, Soline^{®} (cf. la demande internationale WO 01/21150). Cette composition est particulièrement adaptée pour le traitement de l'inflammation et pour la réparation de la barrière cutanée.

Une autre association particulièrement avantageuse selon l'invention est une composition comprenant l'extrait de graines d'*Acacia macrostachya* et un insaponifiable de Soja, tel qu'obtenu selon le procédé décrit dans la demande internationale WO01/51596.

Une autre association particulièrement avantageuse selon l'invention est une composition comprenant l'extrait de graines d'*Acacia macrostachya* et du Lupéol (FR 2 822 821, FR 2 857 596). Cette composition est particulièrement adaptée pour favoriser la cicatrisation.

Une autre association particulièrement avantageuse selon l'invention est une composition comprenant l'extrait de graines d'*Acacia macrostachya* et des peptides de Lupin tels qu'obtenus selon le procédé décrit dans la demande WO2005/102259. Cette composition est particulièrement adaptée pour le traitement de l'inflammation et est utilisée pour ses propriétés anti-âge.

Une autre association particulièrement avantageuse selon l'invention est une composition comprenant l'extrait de graines d'*Acacia macrostachya* et un extrait total de Lupin (voir demande internationale WO2005/102259). Cette composition est particulièrement adaptée pour le traitement des irritations.

Une autre association particulièrement avantageuse selon l'invention est une composition comprenant l'extrait de graines d'*Acacia macrostachya* et une huile de Lupin, avantageusement une huile de Lupin blanc doux, telle que celle décrite dans la demande internationale WO 98/47479.

Une autre association particulièrement avantageuse selon l'invention est une composition comprenant l'extrait de graines d'*Acacia macrostachya* et un extrait peptidique de Maca (voir demande internationale WO2004/112742). Cette composition est particulièrement appréciée pour ses propriétés cicatrisantes et anti-âge.

Une association particulièrement avantageuse selon l'invention est une composition comprenant l'extrait de graines peptidique et osidique d'*Acacia macrostachya* et des peptides de Riz (voir demande internationale WO 2008/009709). Cette composition est particulièrement appréciée pour ses propriétés de stimulation de la mélanogénèse et du transfert de la mélanine.

Une autre association particulièrement avantageuse selon l'invention est une composition comprenant l'extrait de graines d'*Acacia macrostachya* et du Cyclocéramide® (dérivé d'oxazoline) tel que décrit dans les demandes internationales WO2004050052, WO2004050079, et WO2004112741. Cette composition est particulièrement adaptée pour le traitement des réactions inflammatoires.

Une autre association particulièrement avantageuse selon l'invention est une composition comprenant l'extrait de graines d'*Acacia macrostachya* et un extrait de Quinoa, en particulier un extrait peptidique (voir la demande internationale WO2008/080974). Cette composition est particulièrement adaptée pour le traitement des conditions inflammatoires et de la réparation de la barrière cutanée.

Une autre association particulièrement avantageuse selon l'invention est une composition comprenant l'extrait de graines d'*Acacia macrostachya* et un beurre de Cupuaçu. Cette composition est particulièrement appréciée pour ses propriétés hydratantes.

Une autre association particulièrement avantageuse selon l'invention est une composition comprenant l'extrait de graines d'*Acacia macrostachya* et un concentrat de Colza.

Une autre association avantageuse selon l'invention est une composition comprenant l'extrait de graines d'*Acacia macrostachya* et un concentrat de Maïs.

Une autre association avantageuse selon l'invention est une composition comprenant l'extrait de graines d'*Acacia macrostachya* et un extrait de fruit de *Schizandra sphenanthera* (voir demandes françaises FR 0955343 et FR 0955344).

Une autre association avantageuse selon l'invention est une composition comprenant l'extrait de graines d'*Acacia macrostachya* et un extrait de graines de *Vigna unguiculata.*

Toutes ces associations comprennent au moins un autre composé actif, en plus de l'extrait de graines d'*Acacia macrostachya*, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale. Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Selon une deuxième variante, les différentes préparations sont adaptées à une administration orale ; l'extrait de graines d'*Acacia macrostachya* pouvant entrer soit dans un complément alimentaire soit dans une composition nutraceutique. Le complément alimentaire peut se présenter sous forme de l'extrait de graines d'*Acacia macrostachya* en tant que tel ou bien sous forme de gélules ou de capsules molles de gélatine ou végétales dans le cadre de la présente invention. Ledit complément alimentaire peut alors contenir de 10 à 100% en poids de l'extrait de graines d'*Acacia macrostachya.*

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal, comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable, ou un excipient cosmétiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

La composition comprenant un extrait de graines d'*Acacia macrostachya* ayant les spécifications indiquées est particulièrement destinée à une utilisation cosmétique, pharmaceutique, dermatologique ou nutraceutique.

Dans le cadre d'une utilisation cosmétique, pharmaceutique, ou dermatologique, la composition sera avantageusement formulée sous la forme d'une préparation adaptée à une administration topique. La composition comprenant un extrait peptidique et osidique est particulièrement destinée à une utilisation cosmétique, pharmaceutique, ou dermatologique.

Dans le cadre d'une utilisation à visée nutraceutique ou cosmétique (« cosmet-food »), la composition sera avantageusement formulée sous la forme d'une préparation adaptée à une administration orale.

L'invention a également pour objet l'utilisation d'un extrait de graines d'*Acacia macrostachya*, pour la fabrication d'une composition cosmétique, pharmaceutique, dermatologique ou d'une composition nutraceutique.

Avantageusement, la composition ou l'extrait selon la présente invention est utilisée dans la prévention et/ou le traitement des troubles ou pathologies de la peau et/ou des muqueuses et/ou des phanères. De manière particulièrement avantageuse, l'extrait ou la composition selon l'invention est utilisé dans des applications cosmétiques, avantageusement par voie topique, notamment pour le soin ou l'hygiène de la peau et/ou des muqueuses et/ou des phanères tels que les cheveux, ou encore pour la prévention et/ou le traitement des troubles de la peau et/ou des muqueuses et/ou des phanères tels que les cheveux.

La composition ou l'extrait selon la présente invention peut également être utilisée dans la prévention et/ou le traitement des troubles vasculaires.

La composition ou l'extrait selon la présente invention peut également être avantageusement utilisée dans la prévention et/ou le traitement des altérations du tissu adipeux.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

Avantageusement, la composition ou l'extrait selon l'invention peut être utilisé(e) pour la prévention et/ou le traitement des réactions, troubles ou pathologies :
- de la peau, telles que l'acné, la rosacée ou érythrocouperose, le psoriasis, les troubles vasculaires, la dermite du siège, la dermatite atopique, l'eczéma, la dermatite de contact, la dermatite irritative, la dermatite allergique, la dermite séborrhéique (croûte de lait), le psoriasis, la peau sensible, la peau réactive, la peau sèche (xérose), la peau déshydratée, la peau avec rougeur, l'érythème cutané, la peau âgée ou photoâgée, la peau photosensibilisée, la peau pigmentée (mélasma, pigmentation post-inflammatoire...), la peau dépigmentée (vitiligo), la peau avec cellulite, la peau relâchée, la peau avec vergetures, les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutisme, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

L'invention concerne également un procédé de soin cosmétique de la peau et/ou des phanères et/ou des muqueuses, en vue d'améliorer leur état et/ou leur aspect, comprenant l'administration ou consistant à administrer une composition ou un extrait selon la présente invention.

De manière particulièrement avantageuse, la présente invention concerne l'utilisation cosmétique de la composition ou l'extrait pour restaurer les troubles ou désordres de la barrière cutanée, pour renforcer la fonction barrière de la peau, notamment pour lutter contre le stress ou les agressions environnementales ou les agressions ou irritations chimiques provoquées par exemple par les médicaments, sur la peau, les muqueuses ou les phanères, notamment les cheveux.

En particulier, la composition ou l'extrait est utilisé, avantageusement dans des applications cosmétiques, pour hydrater la peau ou les muqueuses, pour traiter les peaux sèches, les peaux atopiques, les peaux agressées, les peaux sensibles, les peaux réactives, les peaux photo-sensibilisées, les peaux ayant subi un traitement anti-acnéique, les peaux âgées ou photo-âgées, de manière générale comme agents antivieillissement de la peau (vieillissement intrinsèque ou extrinsèque), notamment comme agents photo-vieillissement ou agents anti-UV, pour cicatriser la peau, comme agents anti-oxydants et anti-microbiens, ou encore pour le soin des cheveux, des ongles ou des muqueuses.

### EXEMPLES

### Exemple 1 : Compositions pour application par voie topique

Plusieurs compositions pour application par voie topique sont présentées ci-dessous. L'extrait peptidique et osidique d'*Acacia macrostachya* peut être incorporé à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous à titre d'exemples.

**EAU NETTOYANTE HYDRATANTE**

| **Matière première / Nom commercial ou Nom INCI** | **%** |
|---|---|
| EAU PURIFIÉE | QSP 100 % |
| BIOSACCHARID GUM | De 1 à 5 % |
| BUTYLENE GLYCOL | De 1 à 5 % |
| ACIDE HYALURONIQUE | De 0 à 5 % |
| EXTRAIT PEPTIDIQUE ET OSIDIQUE D'ACACIA MACROSTACHYA | De 0,001 à 10 % |
| CONSERVATEURS | De 0 à 1 % |
| ACIDE CITRIQUE MONOHYDRATE | De 0 à 1 % |
| TROMETHAMINE | De 0 à 1 % |

**EAU NETTOYANTE PEAU SENSIBLE**

| **Matière première / Nom commercial ou Nom INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| EXTRAIT D'ACACIA MACROSTACHYA | De 0,001 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

**EMULSION ANTI-AGE**

| **Matière première / Nom commercial ou Nom INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| EXTRAIT D'ACACIA MACROSTACHYA | De 0,01 à 10 % |
| ISONONYL ISONONANOATE | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**EMULSION RESTRUCTURANTE**

| **Matière première / Nom commercial ou Nom INCI** | **%** |
|---|---|
| HYDROGENATED POLYDECENE | De 5 à 20 % |
| LAURYLGLUCOSIDE-GLYSTEARATE | De 1 à 5 % |
| DICAPRYLYL CARBONATE | De 1 à 5 % |
| GLYCEROL | De 5 à 20 % |
| CARBOPOL | De 0 à 1 % |
| GOMME XANTHANE | De 0 à 1 % |
| ACIDE ASIATIQUE | De 0 à 1 % |
| VITAMINE B5 | De 0 à 5 % |
| EXTRAIT D'ACACIA MACROSTACHYA | De 0,01 à 10 % |
| LESSIVE SOUDE | De 0 à 1 % |
| CONSERVATEURS | De 0 à 1 % |
| ACIDE CITRIQUE | De 0 à 1 % |
| EAU PURIFIÉE | QSP 100 % |

**LAIT pour PEAU SECHE, ATOPIQUE**

| **Matière première / Nom commercial ou Nom INCI** | **%** |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5% |
| EXTRAIT D'ACACIA MACROSTACHYA | De 0,1 à 10% |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**STICK ROLL-ON ANTI-BACTERIEN**

| **Matière première / Nom commercial ou Nom INCI** | **%** |
|---|---|
| EAU PURIFIÉE | QSP 100 % |
| BUTYLENE GLYCOL | De 1 à 5 % |
| PEROXYDE DE BENZOYLE | De 0 à 2 % |
| CAPRILOYL GLYCINE | De 0 à 5 % |
| ZINC PCA | De 0 à 5 % |
| EXTRAIT D'ACACIA MACROSTACHYA | De 0,1 à 10 % |
| CARBOMERE | De 0 à 2 % |
| CONSERVATEURS | De 0 à 1 % |
| ACIDE CITRIQUE | De 0 à 1 % |
| TROMETHAMINE | De 0 à 1 % |

**FLUIDE KERATINISANT**

| **Matière première / Nom commercial ou Nom INCI** | **%** |
|---|---|
| CETYL ALCOHOL | De 1 à 5 % |
| SILICONE 345 | De 1 à 5 % |
| ANTI-OXYDANT | De 0 à 1 % |
| EAU PURIFIEE | QSP 100 % |
| CETRIMONIUM CHLORIDE | De 0 à 5 % |
| QUINIE | De 0 à 5 % |
| VITAMINE B5 | De 0 à 5 % |
| EXTRAIT D'ACACIA MACROSTACHYA | De 0,01 à 10 % |
| HYDROLYZED WHEAT PROTEIN | De 0 à 1 % |
| CONSERVATEUR | De 0 à 2 % |
| PARFUM | De 0 à 1 % |
| AJUSTEUR pH | De 0 à 1 % |

**LOTION CAPILLAIRE FORTIFIANTE**

| **Matière première / Nom commercial ou Nom INCI** | **%** |
|---|---|
| EAU PURIFIEE | QSP 100 % |
| METHYL PROPANEDIOL | De 5 à 20% |
| CONSERVATEUR | De 0 à 2 % |
| AJUSTEUR pH | De 0 à 1 % |
| PARFUM | De 0 à 1 % |
| BIOTINE | De 0 à 5 % |
| VITAMINE B9 | De 0 à 5 % |
| EXTRAIT D'ACACIA MACROSTACHYA | De 0,01 à 10 % |
| BETA-SITOSTEROL | De 0 à 1 % |
| ETHYLHEXYL COCOATE | De 0 à 5 % |
| PEG-40 CASTOR OIL | De 0 à 5 % |

**CREME SOLAIRE SPF 50+**

| **Matière première / Nom commercial ou Nom INCI** | **%** |
|---|---|
| EAU PURIFIÉE B4 | QSP 100% |
| OXYDE DE TITANE | De 10 à 20% |
| CYCLOPENTASILOXANE | De 5 à 15% |
| OCTYL PALMITATE | De 5 à 15% |
| C12-C15 ALKYL BENZOATE | De 5 à 10% |
| DECYL PENTANOATE | De 5 à 10% |
| OXYDE DE ZINC | De 5 à 10% |
| GLYCEROL | De 1 à 5% |
| PEG-45/DODECYL GLYCOL COPOLYMERE | De 1 à 5% |
| EXTRAIT D'ACACIA MACROSTACHYA | De 0,01 à 10 % |
| CHLORURE DE SODIUM | De 1 à 5% |
| DEXTRIN PALMITATE | De 1 à 2% |
| VITAMINE E | De 0 à 2% |
| CONSERVATEURS | De 0 à 2% |
| HYDROXYPROPYL GUAR | De 0 à 1% |
| ALOE VERA | De 0 à 1% |
| LESSIVE SOUDE | De 0 à 1% |
| EDTA 2 Na | De 0 à 1% |
| GLUCONATE ZINC | De 0 à 1% |

**VERNIS POUR ONGLES FRAGILES ET CASSANTS**

| **Matière première / Nom commercial ou Nom INCI** | **%** |
|---|---|
| ACRYLATE COPOLYMER | De 15 à 30 % |
| ETHANOL | QSP 100% |
| ACETONE | De 5 à 20 % |
| EXTRAIT D'ACACIA MACROSTACHYA | De 0,01 à 5 % |

### Exemple 2 : Compositions pour administration par voie orale

Les extraits d'*Acacia macrostachya* peuvent être avantageusement intégrés à des compositions orales, typiquement dans des compositions permettant l'administration de 50 mg à 200 mg d'extrait d*'Acacia macrostachya* par jour.

### 1/ Composition anti-vergetures sous forme de capsules molles

| | |
|---|---|
| - EXTRAIT D'ACACIA MACROSTACHYA | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine | QSP 1 capsule molle |

Cette composition est administrée de 4 à 6 capsules de 500 mg par jour.

### 2/ Comprimés anti-chute cheveux

| | |
|---|---|
| - EXTRAIT D'ACACIA MACROSTACHYA | 25 mg |
| - Extraits de céréales (blé, sarrasin, millet, épeautre) riche en acides aminés soufrés | 200 mg |
| - Vitamine C | QSP 50 % des AJR |
| - Glycosaminoglycanes issus de cartilages de poissons | 200 mg |
| - Glucidex IT 19 (agent de compression) | QSP 1 comprimé de 800 mg. |

Cette composition est administrée de 5 à 8 comprimés par jour.

### 3/ Exemples en stick poudre amincissant

| | |
|---|---|
| - EXTRAIT D'ACACIA MACROSTACHYA | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - Ascorbate de sodium | 0,3 mg |
| - Maltodextrine | QSP 5 g. |

Cette composition est administrée 2 fois par jour.

### 4/ Exemples en stick poudre anti-âge

| | |
|---|---|
| **- EXTRAIT D'ACACIA MACROSTACHYA** | 100 mg |
| - Extrait de centella asiatica | 100 mg |
| - Magnesium, sélénium, manganèse | QSP 100 % des AJR. |
| - Gomme xanthane | 1 mg |
| - Ascorbate de sodium | 0,3 mg |
| - Maltodextrine | QSP 5 g. |

Cette composition est administrée 2 fois par jour.

### Exemple 3 : Tests d'activités biologiques de l'extrait selon l'invention

L'extrait peptidique et osidique d'*Acacia macrostachya*, qui est un extrait préparé par hydrolyse telle qu'une hydrolyse enzymatique, est nommé ci-après dans cet exemple hydrolysat d'Acacia.

### I. Screening préliminaire d'activité sur épidermes reconstruits

Les activités biologiques de l'hydrolysat d'Acacia ont été évaluées par un test de modulation d'expression de gènes sur épidermes reconstruits. Ainsi, l'expression de 64 gènes d'intérêts majeurs en physiologie cutanée et cosmétique a été étudiée par PCR-array sur des épidermes reconstruits en cours de différenciation.

### a. Matériel et Méthodes :

L'hydrolysat d'Acacia (0,05% et 0,1%, p/v) a été ajouté dans le milieu de culture d'épidermes reconstruits à J5. Ceux-ci ont alors été incubés pendant 48 heures. L'expression des marqueurs sélectionnés a été évaluée par RT-PCR quantitative (PCR array).

La variation d'expression des marqueurs étudiés par rapport au témoin a été exprimée en pourcentage (Témoin : 100%).

### b. Résultats :

Les résultats les plus significatifs sont présentés dans le tableau ci-dessous et tendent à montrer que l'hydrolysat d'Acacia, en faisant varier l'expression génique de certains marqueurs, a un intérêt particulier notamment dans les activités suivantes :
- **La cicatrisation :** ↗ CLSP, cathélicidine, S100A7, Heme oxygénase 1, MMP9.
- **L'hydratation et la fonction barrière :** ↗ Claudine 1, Desmogléine 1, Glucocérébrosidasee, Loricrine, Small proline-rich proteins, Transglutaminase 1.
- **Les défenses anti-microbiennes et anti-oxydantes :** ↗ Heme oxygénase 1, Hsp27, hBD2, RNase 7, Cathélicidine.
- **Le cheveu :** ↗ kératine 6.

*Variations de l'expression de gènes d'intérêt dans des épidermes reconstruits.*

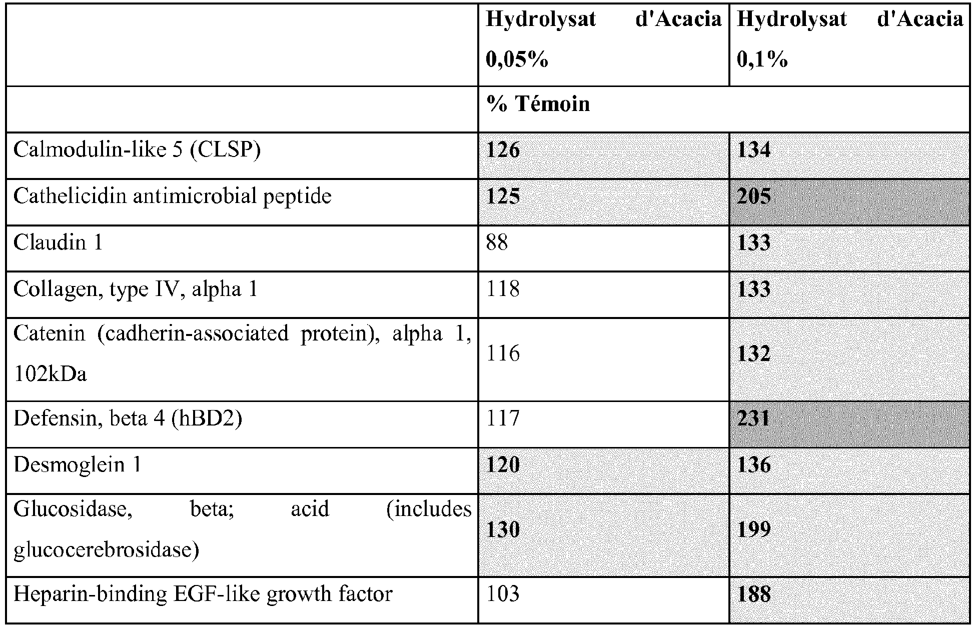

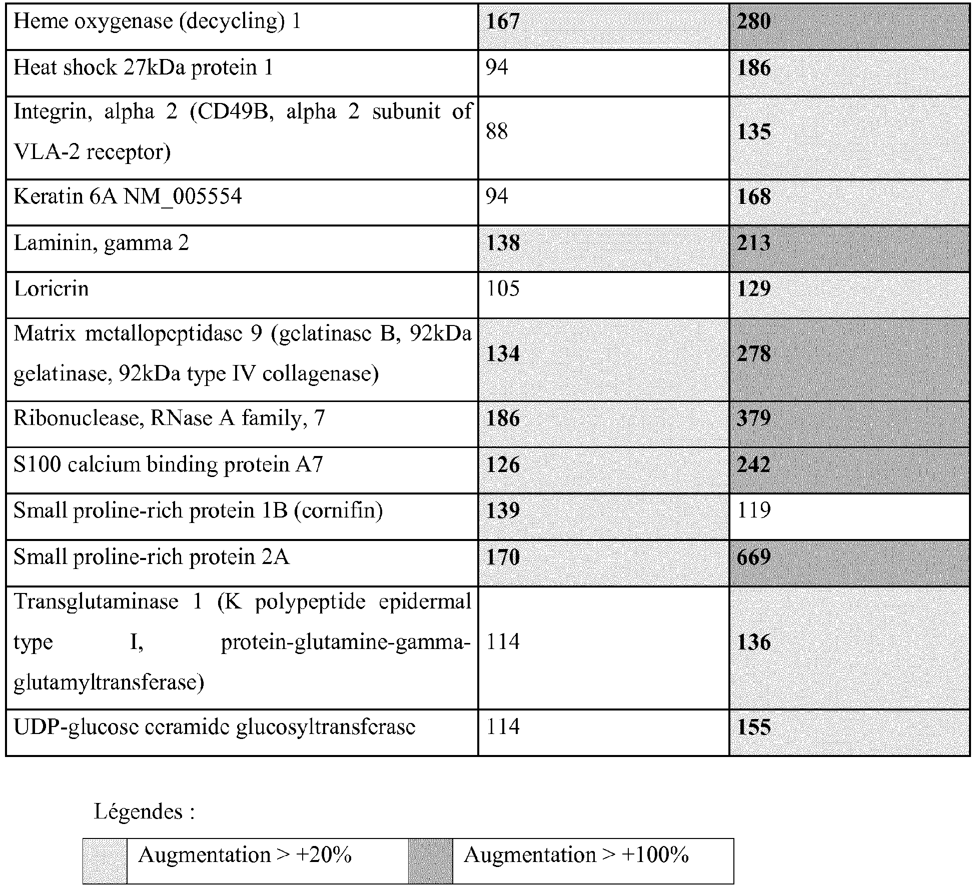

### c. Conclusion :

Ainsi, à l'issue de ce screening, l'activité de l'hydrolysat d'Acacia a été évaluée dans deux axes d'intérêt en dermo-cosmétique : **la barrière et l'hydratation épidermique,** ainsi que **la matrice dermique et le stress oxydant.**

### II. Fonction barrière et hydratation

### A. Introduction

La mise en place de la fonction barrière est liée à la différenciation épidermique qui aboutit à la formation du *stratum corneum* (SC). Différentes structures permettent au SC d'assurer sa fonction de barrière : les lipides intercellulaires, l'enveloppe cornifiée, les cornéodesmosomes.

Cette barrière est renforcée par une seconde ligne de défense située dans le *stratum granulosum* et constituée par les jonctions serrées.

### B. Evaluation de l'expression génique des marqueurs de la différenciation et de la barrière

### a. Matériel et Méthodes :

Des kératinocytes humains normaux ont été traités pendant 24 ou 48 heures par l'hydrolysat d'Acacia à 0,01 ou 0,05% (p/v) ; en parallèle des kératinocytes témoins ont été cultivés en conditions favorables à la différenciation (milieu supplémenté en calcium « High Ca »). L'expression génique des marqueurs sélectionnés a été analysée par RT-PCR quantitative en temps réel.

Les résultats ont été analysés statistiquement par une analyse de variance (ANOVA) à un facteur suivie d'un test de Dunnett :
ns p>0,05 : non significatif
* 0,01<p<0,05 : significatif
** 0,001<p<0,01 : très significatif
***p<0,001 : hautement significatif

Le niveau d'expression génique a été exprimé en quantité relative (QR) et l'effet du traitement par rapport aux cellules contrôles en pourcentage d'augmentation.

### b. Résultats :

L'hydrolysat d'Acacia a très fortement et significativement augmenté l'expression génique de la kératine 1 et de la kératine 10. Par ailleurs, l'hydrolysat d'Acacia a significativement augmenté l'expression génique de la filaggrine, de l'involucrine, de la loricrine et de la transglutaminase 1 (TGase 1).

Toutes ces protéines sont impliquées dans la **formation de l'enveloppe cornée.**

L'hydrolysat d'Acacia contribue donc à renforcer l'enveloppe cornée et donc à améliorer la résistance de la barrière épidermique.

De plus, la filaggrine, en tant que précurseur du NMF (Facteur Naturel d'Hydratation) est impliquée dans **l'hydratation de l'épiderme.** Ainsi, par une action sur la filaggrine, l'hydrolysat d'Acacia contribue à améliorer l'hydratation de l'épiderme.

L'hydrolysat d'Acacia a augmenté significativement l'expression génique de la desmogléine 1 (Dsg 1) et de la desmocolline-1 (Dsc 1), **protéines constitutives des cornéodesmosomes,** cet effet est donc en faveur d'un renforcement de la cohésion du *stratum corneum*.

*Expression génique de marqueurs de la différenciation et de la carrière par des kératinocytes.*

| | Cellules contrôles | **Hydrolysat d'Acacia 0,01%** | **Hydrolysat d'Acacia 0,05%** | Témoin High Ca |
|---|---|---|---|---|
| **KERATINE 1** | 1,00 | **2,29 (+129% *)** | **4,43 (+343% ***)** | **5,42 (+442% ***)** |
| **KERATINE 10** | 1,00 | **2,28 (+128% *)** | **3,52 (+252% ***)** | **8**,**95 (+795% ***)** |
| **FILAGGRINE** | 1,00 | 1,01 (+1% ns) | **1,64 (+64% **)** | **4,11 (+311% ***)** |
| **INVOLUCRINE** | 1,00 | **1,81 (+81% ***)** | 1,18 (+18% ns) | 1,16 (+16>% ns) |
| **LORICRINE** | 1,00 | 1,54 (+54% ns) | **3,53 (+253% **)** | 0,98 (-2% ns) |
| **TGase 1** | 1,00 | **1,60 (+60% *)** | 1,40 (+40% ns) | **2,25 (+125% ***)** |
| **Dsg1** | 1,00 | 1,39 (+39% ns) | **1,49 (+49% *)** | **3,25 (+225% ***)** |
| **Dsc1** | 1,00 | **1,57 (+57%*)** | 1,38 (+38% ns) | **2,70 (+170% ***)** |

### C. Evaluation de l'expression génique des protéines de jonctions

### 1. Screening d'expression des messagers de protéines de jonctions

### a. Matériel et Méthodes :

L'activité de l'hydrolysat d'Acacia a été étudiée par RT-PCR quantitative sur le niveau d'expression de 16 gènes codant principalement pour des protéines des jonctions serrées ainsi que pour des protéines majeures des jonctions communicantes de l'épiderme.

Des kératinocytes humains normaux ont été traités ou non (témoin) par l'hydrolysat d'Acacia à 0,05% et 0,1% (p/v) ou la référence (chlorure de calcium CaCl2 à 1,5 mM) pendant 24 heures. L'expression des marqueurs a été évaluée par RT-PCR quantitative (PCR array).

La variation d'expression des marqueurs étudiés par rapport au témoin a été exprimée en pourcentage (Témoin : 100%).

Les niveaux d'expression sont classifiés de la manière suivante :

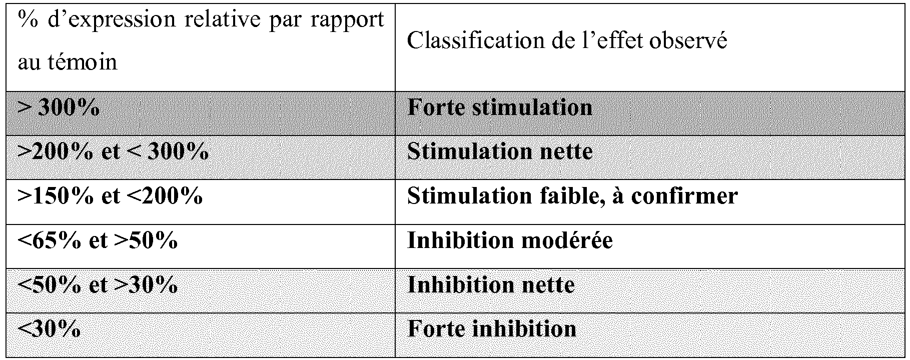

### b. Résultats :

Les résultats présentés ci-dessous montrent que l'hydrolysat d'Acacia a eu un effet similaire au contrôle positif sur l'expression des messagers des jonctions : en effet, l'hydrolysat d'Acacia a entraîné une augmentation significative d'expression des marqueurs de jonctions serrées Claudine 1, Occludine et Cinguline. Une inhibition de l'expression de la Gap Junction Protein β2 a également été observée. Le marqueur de jonction communicante Gap Junction Protein β2 code pour la protéine connexine 26 qui est plus particulièrement exprimée dans les kératinocytes en prolifération.

Ainsi, l'hydrolysat d'Acacia, en stimulant l'expression des marqueurs de jonctions serrées et en inhibant l'expression du marqueur de jonction communicante Gap Junction Protein β2, a présenté un effet pro-différenciant ainsi qu'un effet positif au niveau du **renforcement de la barrière cutanée** permettant de limiter les pertes en eau ainsi qu'en solutés à travers les espaces para-cellulaires.

### Expression des messagers des protéines de jonctions (% du témoin)

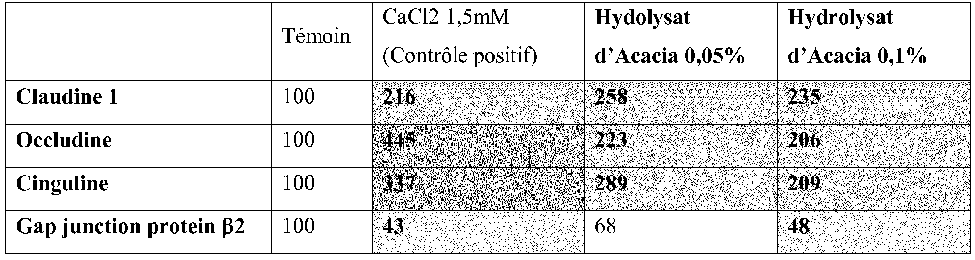

### 2. Evaluation de l'expression génique des marqueurs des jonctions serrées

### a. Matériel et Méthodes :

Des kératinocytes humains normaux ont été traités pendant 24 ou 48 heures par l'hydrolysat d'Acacia à 0,05% ou 0,1% (p/v) ; en parallèle des kératinocytes témoins ont été cultivés en conditions favorables à la différenciation (milieu supplémenté en calcium « High Ca »). L'expression génique des marqueurs des jonctions serrées a été analysée par RT-PCR quantitative en temps réel.

Les résultats ont été analysés statistiquement par une analyse de variance (ANOVA) à un facteur suivie d'un test de Dunnett :
ns p>0,05 : non significatif
* 0,01< p<0,05 : significatif
** 0,001< p<0,01 : très significatif
***p<0,001 : hautement significatif

Le niveau d'expression génique a été exprimé en quantité relative (QR) et l'effet du traitement par rapport aux cellules contrôles en pourcentage d'augmentation.

### b. Résultats :

L'hydrolysat d'Acacia a significativement augmenté l'expression génique de la claudine-1 et de l'occludine, confirmant ainsi les résultats obtenus lors de l'étude précédente en PCR array. Par cette action, et en plus de son action sur la filaggrine, aide donc à maintenir l'**hydratation** de l'épiderme en limitant les pertes en eau.

**Expression génique des marqueurs des jonctions serrées par des kératinocytes.**

| | Cellules contrôles | **Hydrolysat d'Acacia 0,05%** | **Hydrolysat d'Acacia 0,1%** | Témoin High Ca |
|---|---|---|---|---|
| **CLAUDINE 1** | 1,00 | **1,86 (+86% *)** | **2,26 (+126% **)** | **3,22 (+222% ***)** |
| **OCCLUDINE** | 1,00 | 1,41 (+41% ns) | **1,77 (+77% *)** | 2,03 (+103% ns) |

### D. Conclusion

Un effet positif de l'extrait d'Acacia selon la présente invention sur les différentes structures impliquées dans la mise en place de la fonction barrière a été mis en évidence : en effet, l'extrait d'Acacia est capable de réguler positivement l'expression des marqueurs de l'enveloppe cornée, des cornéodesmosomes et des jonctions serrées. Ces propriétés permettent à l'extrait (hydrolysat) d'Acacia de contribuer à la solidité et l'étanchéité de la fonction barrière tout en participant au maintien d'un bon niveau d'hydratation.

### III. Matrice dermique et stress oxydant

### A. Introduction

Les modifications de la peau avec l'âge résultent de modifications des fonctions cellulaires et de modifications progressives de la composition et de la structure de la matrice extra-cellulaire dermique. En effet, le derme subit une perte graduelle de son épaisseur avec une diminution de macromolécules essentielles, comme le collagène et l'élastine. Cette diminution peut être attribuée à une diminution de leur synthèse et/ou à une augmentation de leur dégradation par les MMP (métalloprotéinases matricielles) par exemple.

La relation entre stress oxydant et vieillissement est aujourd'hui bien établie. En effet, il y a une accumulation dans les cellules âgées de molécules endommagées conduisant à une altération des fonctions cellulaires. Pour se protéger contre ce stress oxydatif, la cellule dispose de différents mécanismes de défenses anti-oxydants dont fait partie l'enzyme microsomale heme-oxygénase.

L'effet de l'hydrolysat d'Acacia sur la modulation de la matrice extra-cellulaire du derme et sur la stimulation des défenses anti-oxydantes a été évalué sur un modèle de fibroblastes dermiques sur lesquels l'expression génique de l'élastine, de la MMP1 et de l'heme oxygénase 1 a été étudiée par RT-PCR quantitative en temps réel.

### B. Matériel et méthodes

Des fibroblastes humains normaux ont été traités pendant 24h par l'hydrolysat d'Acacia à 0,01% et 0,05% (p/v) ou par la référence TGFβ1 à 5 ng/ml. L'expression génique des marqueurs sélectionnés a été analysée par RT-PCR quantitative en temps réel.

Les résultats ont été analysés statistiquement par une analyse de variance (ANOVA) à un facteur suivie d'un test de Dunnett :
ns p>0,05 : non significatif
* 0,01< p<0,05 : significatif
** 0,001< p<0,01 : très significatif
***p<0,001 : hautement significatif

Le niveau d'expression génique a été exprimé en quantité relative (QR) et l'effet du traitement par rapport aux cellules contrôles en pourcentage d'augmentation ou d'inhibition.

### C. Résultats

L'hydrolysat d'Acacia a nettement et significativement stimulé l'expression génique de l'élastine et a très fortement et significativement inhibé l'expression génique de la MMP1.

Ainsi, l'hydrolysat d'Acacia pourrait contribuer à **limiter les altérations du derme liées à l'âge.**

Par ailleurs, l'hydrolysat d'Acacia a significativement stimulé l'expression génique de l'heme oxygénase 1, contribuant ainsi à renforcer les **mécanismes de défenses anti-oxydantes** de la cellule.

*Expression génique de marqueurs de la matrice dermique et des défenses anti-oxydantes par des fibroblastes.*

| | **ELASTINE** | **MMP1** | **HEME OXYGENASE 1** |
|---|---|---|---|
| Cellules contrôles | 1,00 | 1,00 | 1,00 |
| Témoin (TGFβ1) | **6,59 (+559% ***)** | **0,12 (-88% ***)** | *non déterminé* |
| **Hydrolysat d'Acacia 0,01%** | **1,76 (+76% **)** | **0,36 (-64% ***)** | 1,22 (+22% ns) |
| **Hydrolysat d'Acacia 0,05%** | **1,52 (+52% *)** | **0,43 (-57% ***)** | **2,35 (+135% ***)** |

### E. Conclusion

Avec l'âge, la matrice extra-cellulaire dermique s'altère, contribuant à l'amincissement du derme.

En plus de ce mécanisme physiologique, d'autres facteurs extrinsèques peuvent contribuer à accélérer le processus de vieillissement cutané : ainsi, les radicaux libres oxygénés, produits sous l'influence des UV par exemple, peuvent altérer les différents composants de la cellule. Pour se défendre, la peau a développé un système de défense anti-oxydant complexe, mais l'accumulation de radicaux libres peut submerger ce système de défense et mener à un déséquilibre ayant tendance à aboutir à un état pro-oxydant, qui à long terme aboutira au photo-vieillissement de la peau.

L'heme oxygénase 1 fait partie de ce système de défense, ainsi en stimulant son expression, l'extrait (hydrolysat) d'Acacia selon l'invention contribue à renforcer le système de défense et de protection de la cellule vis-à-vis du stress oxydant.

De plus, en activant la synthèse d'élastine et en limitant l'expression de la MMP1, l'extrait (hydrolysat) d'Acacia selon l'invention contribue à protéger la cellule des altérations liées au vieillissement, qu'il soit intrinsèque ou extrinsèque.

## Revendications

1. Composition comprenant un extrait de graines *d'Acacia macrostachya* et le cas échéant un excipient approprié, **caractérisée en ce que** l'extrait de graines est un extrait peptidique et osidique substantiellement débarrassé de toute protéine native résiduelle.

2. Composition selon la revendication 1, **caractérisé en ce que** l'extrait peptidique et osidique comprend 10 à 90% en poids de peptides et 10 à 90% en poids de sucres, les pourcentages étant exprimés par rapport au poids total dudit extrait peptidique et osidique.

3. Composition selon la revendication 2, **caractérisé en ce que** l'extrait peptidique et osidique comprend 20 à 70%, avantageusement 30 à 65%, typiquement 45% à 50%, en poids de peptides.

4. Composition selon la revendication 2 ou 3, **caractérisé en ce que** l'extrait peptidique et osidique comprend 20 à 60%, avantageusement 30 à 55%, typiquement 45% à 50%, en poids de sucres.

5. Composition selon l'une quelconque des revendications précédentes, comprenant au moins un autre composé actif en plus de l'extrait de graines *d'Acacia macrostachya.*

6. Composition selon l'une quelconque des revendications précédentes, **caractérisée en ce qu'**il s'agit d'une composition cosmétique, pharmaceutique, dermatologique ou nutraceutique.

7. Composition selon l'une quelconque des revendications précédentes, **caractérisée en ce qu'**elle est formulée pour être administrée par voie topique ou orale.

8. Procédé de préparation d'un extrait peptidique et osidique des graines *d'Acacia macrostachya* substantiellement débarrassé de toute protéine native résiduelle, comprenant les étapes successives suivantes :
- dispersion en phase aqueuse des graines broyées, avantageusement à un pH compris entre 3.0 et 9.0 et à une température comprise entre 20 et 90°C,
- hydrolyse enzymatique de ladite dispersion, et
- récupération de l'extrait peptidique et osidique.

9. Procédé selon la revendication 8, **caractérisé en ce que** l'hydrolyse est une hydrolyse enzymatique réalisée par un mélange de protéases et de carbohydrases, telles que des pectinases, cellulases, arabanases, hémicellulases, et β-glucanases.

10. Procédé selon la revendication 8 ou 9, **caractérisé en ce que**, suite à l'hydrolyse de ladite dispersion, et préalablement à la récupération de l'extrait peptidique et osidique, on procède à une étape d'ultrafiltration par exemple à un seuil de coupure compris entre 10000 et 15000 Daltons.

11. Extrait de graines *d'Acacia macrostachya* substantiellement débarrassé de toute protéine native résiduelle susceptible d'être obtenu par le procédé selon l'une quelconque des revendications 8 à 10, contenant 10 à 90% en poids de peptides et 10 à 90% en poids de sucres.

12. Extrait selon la revendication 11, contenant 20 à 70%, avantageusement 30 à 65%, typiquement 45% à 50%, en poids de peptides.

13. Extrait selon la revendication 11 ou 12, contenant 20 à 60%, avantageusement 30 à 55%, typiquement 45% à 50%, en poids de sucres.

14. Extrait selon l'une des revendications 11 à 13 ou composition selon l'une quelconque des revendications 1 à 7 pour son utilisation dans la prévention et/ou le traitement des troubles ou pathologies de la peau et/ou des muqueuses et/ou des phanères.

15. Extrait selon l'une des revendications 11 à 13 ou composition selon l'une quelconque des revendications 1 à 7 pour son utilisation dans la prévention et/ou le traitement des altérations du tissu adipeux.

16. Procédé de soin cosmétique de la peau et/ou des phanères et/ou des muqueuses, en vue d'améliorer leur état et/ou leur aspect, comprenant l'administration d'une composition cosmétique telle que définie selon l'une quelconque des revendications 1 à 7 ou d'un extrait tel que défini selon l'une quelconque des revendications 11 à 13.

17. Procédé de soin cosmétique selon la revendication 16, pour hydrater la peau et les muqueuses ou pour le traitement des peaux sèches.

## Patentansprüche

1. Zusammensetzung, die einen Extrakt der Saaten von *Acacia macrostachya* und, falls notwendig, einen geeigneten Hilfsstoff enthält, **dadurch gekennzeichnet, dass** der Saatextrakt ein Peptid und Osidextrakt ist, der wesentlich allem restlichen nativen Protein befreit ist.

2. Zusammensetzung nach Anspruch 1, **dadurch gekennzeichnet, dass** das Peptid und Osidextrakt 10-90 Gew-% Peptide und 10-90 Gew-% Zucker umfasst, wobei die Prozentsätze in Bezug auf dieses Gesamtgewicht des Peptids und Osidextrakts ausgedrückt sind.

3. Zusammensetzung nach Anspruch 2, **dadurch gekennzeichnet, dass** das Peptid und Osidextrakt 20-70 %, vorteilhaft 30-65 %, typisch 45-50 Gew-% der Peptide umfasst.

4. Zusammensetzung nach Anspruch 2 oder 3, **dadurch gekennzeichnet, dass** das Peptid und Osidextrakt 20-60 %, vorteilhaft 30-55 %, typisch 45-50 Gew-% der Zucker umfasst.

5. Zusammensetzung nach einem der vorherigen Ansprüche, die mindestens eine weitere aktive Verbindung zusätzlich zum *Acacia macrostachya-*Saatextrakt enthält.

6. Zusammensetzung nach einem der vorherigen Ansprüche, **dadurch gekennzeichnet, dass** sie eine kosmetische, pharmazeutische, dermatologische oder nutrazeutische Zusammensetzung ist.

7. Zusammensetzung nach einem der vorherigen Ansprüche, **dadurch gekennzeichnet, dass** sie so formuliert ist, dass sie topisch oder oral verabreicht werden kann.

8. Verfahren zum Herstellen eines Peptids und Osidextrakts aus *Acacia macrostachya*-Saaten die im Wesentlichen von allem restlichen nativen Proteinen befreit sind, das folgenden aufeinanderfolgenden Schritte umfasst:
- Dispersion in der wässrigen Phase der gemahlenen Saaten, vorteilhafterweise bei einem pH, der zwischen 3,0 und 9,0 liegt, und bei einer Temperatur, die zwischen 20 und 90 °C liegt,
- enzymatische Hydrolyse der Dispersion, und
- Rückgewinnung des Peptids und Osidextrakts.

9. Verfahren nach Anspruch 8, **dadurch gekennzeichnet, dass** die Hydrolyse eine enzymatische Hydrolyse ist, die von einer Mischung von Proteasen und Carbohydrasen ausgeführt wird, wie zum Beispiel Pectinasen, Cellulasen, Arabanasen, Hemicellulasen und β-Glucanases.

10. Verfahren nach Anspruch 8 oder 9, **dadurch gekennzeichnet, dass** nach der Hydrolyse der Dispersion und vor der Rückgewinnung des Peptids und Osidextrakts ein Schritt der Ultrafiltration ausgeführt wird, zum Beispiel an einem Trennpunkt, der zwischen 10.000 und 15.000 Dalton liegt.

11. *Acacia macrostachya*-Saatextrakt, im Wesentlichen befreit von allem restlichen nativen Protein, erreichbar durch das Verfahren nach einem der Ansprüche 8-10, 10-90 Gew-% Peptide und 10-90 Gew-% Zucker umfasst.

12. Extrakt nach Anspruch 11, der 20-70 %, vorteilhafterweise 30-65 %, typisch 45-50 Gew-% Peptide enthält.

13. Extrakt nach Anspruch 11 oder 12, der 20-60 %, vorteilhafterweise 30-55 %, typisch 45-50 Gew-% Zucker enthält.

14. Extrakt nach einem der Ansprüche 11-13 oder Zusammensetzung nach einem der Ansprüche 1-7 zur Verwendung in der Prävention und/oder der Behandlung von Störungen oder Krankheiten, die die Haut und/oder Schleimhäute und/oder die Anhangsgebilde beeinträchtigen.

15. Extrakt nach einem der Ansprüche 11-13 oder Zusammensetzung nach einem der Ansprüche 1-7 zur Verwendung in der Prävention und/oder der Behandlung von Veränderungen des Fettgewebes.

16. Verfahren zur kosmetischen Pflege der Haut und/oder der Haut-Anhangsgebilde und/oder der Schleimhäute, um den Zustand und/oder das Aussehen derselben zu verbessern, das die Verabreichung einer kosmetischen Zusammensetzung wie nach einem der Ansprüche 1-7 definiert, oder eines Extrakts, wie nach einem der Ansprüche 11-13 definiert, umfasst.

17. Verfahren zur kosmetischen Pflege nach Anspruch 16, zum Befeuchten der Haut und der Schleimhäute oder für die Behandlung von trockener Haut.

## Claims

1. Composition comprising an extract of seeds of *Acacia macrostachya* and if necessary an appropriate excipient, **characterized in that** the seed extract is a peptide and oside extract substantially cleared of any residual native protein.

2. Composition according to claim 1, **characterized in that** the peptide and oside extract comprises 10 to 90% by weight of peptides and 10 to 90% by weight of sugars, the percentages being expressed in relation to the total weight of said peptide and oside extract.

3. Composition according to claim 2, **characterized in that** the peptide and oside extract comprises 20 to 70%, advantageously 30 to 65%, typically 45% to 50%, by weight of peptides.

4. Composition according to claim 2 or 3, **characterized in that** the peptide and oside extract comprises 20 to 60%, advantageously 30 to 55%, typically 45% to 50%, by weight of sugars.

5. Composition according to any one of the preceding claims, comprising at least one other active compound in addition to the *Acacia macrostachya* seed extract.

6. Composition according to any one of the preceding claims, **characterized in that** it is a cosmetic, pharmaceutical, dermatological or nutraceutical composition.

7. Composition according to any one of the preceding claims, **characterized in that** it is formulated to be administered by topical or oral route.

8. Method for preparing a peptide and oside extract of *Acacia macrostachya* seeds substantially cleared of any residual native protein, comprising the following successive steps:
- dispersion in aqueous phase of the ground seeds, advantageously at a pH comprised between 3.0 and 9.0 and at a temperature comprised between 20 and 90°C,
- enzymatic hydrolysis of said dispersion, and
- recovery of the peptide and oside extract.

9. Method according to claim 8, **characterized in that** the hydrolysis is an enzymatic hydrolysis carried out by a mixture of proteases and carbohydrases, such as pectinases, cellulases, arabanases, hemicellulases, and β-glucanases.

10. Method according to claim 8 or 9, **characterized in that**, following the hydrolysis of said dispersion, and prior to the recovery of the peptide and oside extract, a step of ultra-filtration is carried out for example at a cut-off point comprised between 10000 and 15000 Daltons.

11. *Acacia macrostachya* seed extract substantially cleared of any residual native protein obtainable by the method according to any one of claims 8 to 10, containing 10 to 90% by weight of peptides and 10 to 90% by weight of sugars.

12. Extract according to claim 11, containing 20 to 70%, advantageously 30 to 65%, typically 45% to 50%, by weight of peptides.

13. Extract according to claim 11 or 12, containing 20 to 60%, advantageously 30 to 55%, typically 45% to 50%, by weight of sugars.

14. Extract according to one of claims 11 to 13 or composition according to any one of claims 1 to 7 for its use in the prevention and/or the treatment of disorders or diseases affecting the skin and/or the mucosae and/or the appendages.

15. Extract according to one of claims 11 to 13 or composition according to any one of claims 1 to 7 for its use in the prevention and/or the treatment of the adipose tissue alterations.

16. Method of cosmetic care of the skin and/or the appendages and/or the mucosae, in order to improve the condition and/or the appearance thereof, comprising the administration of a cosmetic composition as defined according to any one of claims 1 to 7 or of an extract as defined according to any one of claims 11 to 13.

17. Method of cosmetic care according to claim 16, for moisturising the skin and the mucosae or for the treatment of dry skins.
